(19) Europäisches Patentamt
European Patent Office
Office européen des brevets

(11) Veröffentlichungsnummer: **0 149 222**
**B1**

(12) # EUROPÄISCHE PATENTSCHRIFT

(45) Veröffentlichungstag der Patentschrift: **07.11.90**

(21) Anmeldenummer: **84116169.8**

(22) Anmeldetag: **22.12.84**

(51) Int. Cl.$^5$: **C 07 J 5/00,** C 07 J 7/00,
A 61 K 31/57 // C07J21/00

(54) **Neue 6alpha, 16alpha-Dimethylkortikoide.**

(30) Priorität: **02.01.84 DE 3400188**

(43) Veröffentlichungstag der Anmeldung:
**24.07.85 Patentblatt 85/30**

(45) Bekanntmachung des Hinweises auf die Patenterteilung:
**07.11.90 Patentblatt 90/45**

(84) Benannte Vertragsstaaten:
**AT BE CH DE FR GB IT LI LU NL SE**

(56) Entgegenhaltungen:
**GB-A- 898 292**
**GB-A- 921 534**
**US-A-3 004 994**
**US-A-3 290 338**
**US-A-3 350 426**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Annen, Klaus. Dr.**
**Osthofstrasse 80**
**D-4400 Münster-Albachten (DE)**
Erfinder: **Laurent, Henry. Dr.**
**Glambecker Weg 21**
**D-1000 Berlin 28 (DE)**
Erfinder: **Hofmeister, Helmut. Dr.**
**Weislingenstrasse 4**
**D-1000 Berlin 28 (DE)**
Erfinder: **Töpert, Michael. Dr.**
**Sigismundkorso 58**
**D-1000 Berlin 28 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 149 222 B1

**Beschreibung**

Die Erfindung betrifft neue 6α,16α-Dimethylkortikoide der allgemeinen Formel I

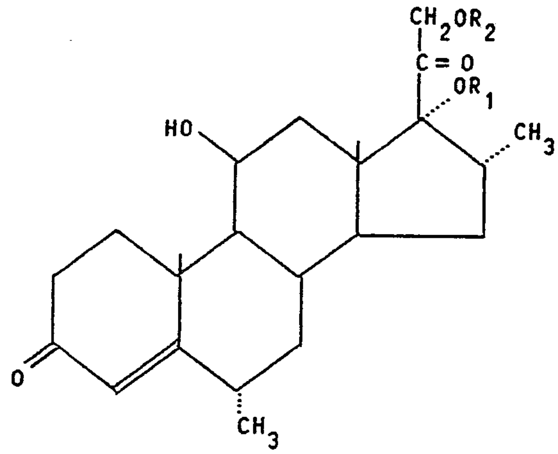

worin

die Bindung ...... eine Einfachbindung oder eine Doppelbindung darstellt, $R_1$ eine 2 bis 6 Kohlenstoffatome enthaltende Alkanoylgruppe bedeutet und $R_2$ ein Wasserstoffatom oder eine Alkanoylgruppe mit 2 bis 6 Kohlenstoffatomen bedeutet.

Die neuen 6,16-Dimethylkortikoide können als Substituenten $R_1$ eine 2 bis 6 Kohlenstoffatome enthaltende Alkanoylgruppe enthalten. Geeignete Alkanoylgruppen $R_1$ sind beispielsweise die Acetylgruppe, Propionylgruppe, die Butyrylgruppe, die Isobutyrylgruppe, die Valerianylgruppe, die 3-Methylbutyrylgruppe, die Trimethylacetylgruppe oder die Hexanoylgruppen.

Als Substituenten $R_2$ können die 6,16-Dimethylkortikoide ein Wasserstoffatom, oder eine 2 bis 6 Kohlenstoffatome enthaltende Alkanoylgruppe enthalten. Geeignete Substituenten sind beispielsweise die bereits erwähnten Alkanoylgruppen.

Die 6,16-Dimethylkortikoide der allgemeinen Formel I gemäß Patentanspruch 1, zeichnen sich bei topischer Applikation durch eine ausgeprägte antiinflammatorische Wirksamkeit aus. Darüberhinaus zeigen sie eine ausgezeichnete Dissoziation zwischen erwünschter topischer Wirksamkeit und unerwünschten systemischen Nebenwirkungen. Sie übertreffen hierin diejenigen Kortikoide welche beispielsmäßig in der GB—A 898,292, der GB—A 2,921,534, der US—A 3,004,994, der US—A 3,290,338 und der US—A 3,350,426 beschrieben sind.

Die neuen 6,16-Dimethylkortikoide der allgemeinen Formel I gemäß Patentanspruch 1 eignen sich in Kombination mit den in der galenischen Pharmazie üblichen Trägermitteln zur lokalen Behandlung von Kontaktdermatitis, Ekzemen der verschiedensten Art, Neurodermatosen, Erythrodermie, Ver- brennungen, Pruritis vulvae et ani, Rosacea, Erythematodes cutaneus, Psoriasis, lichen ruber planus et verrucosus und ähnlichen Hauterkrankungen.

Die Herstellung der Arzneimittelspezialitäten erfolgt in üblicher Weise, indem man die Wirkstoff mit geeigneten Zusätzen in die gewünschte Applikationsform, wie zum Beispiel: Lösungen, Lotionen, Salben, Cremes oder Pflaster, überführt. In den so formulierten Arzneimitteln ist die Wirkstoffkonzentration von der Applikationsform abhängig. Bei Lotionen und Salben wird vorzugsweise eine Wirkstoffkonzentration von 0,001% bis 1% verwendet.

Darüberhinaus sind die neuen Verbindungen gegebenenfalls in Kombination mit den üblichen Trägermitteln und Hilfstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Die neuen 6,16-Dimethylkortikoide können unter den Bedingungen hergestellt werden, wie sie in den deutschen Patentanmeldungen Nr. P 26 45 105.8 und 28 03 661.5 beschrieben sind.

Die nachfolgenden Ausführungsbeispiele dienen zur Erläuterung der Erfindung.

Beispiel 1

a) Aus dem trüben Gemisch aus 1 g 9-Fluor-11β,17,21-Trihydroxy-6α,16α-dimethyl-4-pregnen-3,20-dion und Pyridiniumtosylat in 9 ml Dimethylformamid und 80 ml Hexan werden bei 130°C Badtemperatur ca. 30 ml lösungsmittel abdestilliert. Nach kurzem Abkühlen gibt man in die warme Lösung 2 ml Orthopropionsäuretriethylester und destilliert das restliche Hexan ab. Schließlich wird die Lösung mit ml Pyridin versetzt und im Vakuum bei 60°C eingeengt. Man erhält 17,21-(1-Ethoxy-propylidendioxy-11β-hydroxy-6α,16α-dimethyl-4-pregnen-3,20-dion als Öl.

b) Eine Suspension des rohen 17,21-(1-Ethoxy-propylidendioxy-11β-hydroxy-6α,16α-dimethyl-4-pregnen-3.20-dion in 24 ml Methanol wird mit einem Gemisch aus 1.1 ml 0.1M wäßriger Natriumacetat-Lösung und 10 ml 0.1N wäßriger Essigsäure 1 Stunde bei 100°C refluxiert. Man engt bis zur Trübung ein, gibt auf Wasser und extrahiert mit Essigester. Die organische Phase wird mit Wasser gewaschen, über Natriumsulfat getrocknet und im Vakuum zur Trockne eingedampft. Des Rohprodukt wird an 90 g Kieselgel mit einem Hexan-Aceton-Gradientien (0—50% Aceton) gereinigt. Ausbeute 783 mg 17α-Propyloxy-11β,21-dihydroxy-6α,16α-dimethyl-4-pregnen-3,20-dion.

Beispiel 2

Eine Lösung von 2.0 g 21-Acetoxy-11β-hydroxy-6α,16α-dimethyl-17-propionyloxy-4-pregnen-3,20-dion in 100 ml Dioxan wird mit 2.0 g Dichlorodicyanobenzochinon 24 Stunden am Rückfluß gekocht. Nach dem Abkühlen wird der Feststoff abgesaugt, mit Hexan gewaschen und das Filtrat im Vakuum eingeengt. Das Rohprodukt wird an Kieselgel mit einem Hexan-Aceton-Gradienten (0—40% Aceton) gereinigt. Man isoliert 1.05 g 21-Acetoxy-11β-hydroxy-6α,16α-dimethyl-17-propionyloxy-1,4-pregnadien-3,20-dion. Schmp. 182—184°C.

**Patentansprüche**

1. 6α,16α-Dimethylkortikoide der allgemeinen Formel I

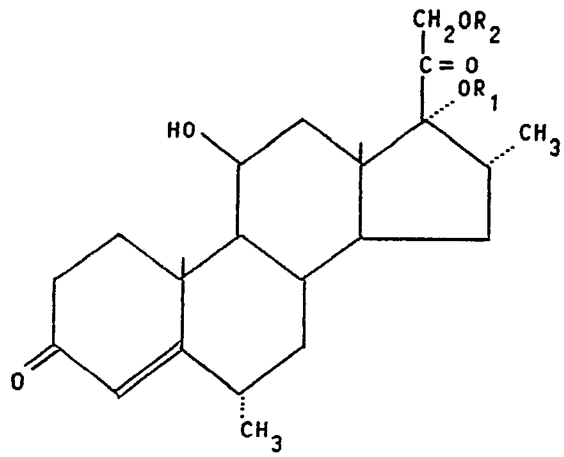

die Bindung ...... eine Einfachbindung oder eine Doppelbindung darstellt, $R_1$ eine 2 bis 6 Kohlenstoffatome enthaltende Alkanoylgruppe bedeutet und $R_2$ ein Wasserstoffatom oder eine Alkanoylgruppe mit 2 bis 6 Kohlenstoffatomen bedeutet.

2. 11β-21-Dihydroxy-6α,16α-dimethyl-17α-propionyloxy-1,4-pregnen-3,20-dion.

3. 21-Acetoxy-11β-hydroxy-6α,16α-dimethyl-17α-propionyloxy-4-pregnen-3,20-dion.

4. 21-Acetoxy-11β-hydroxy-6α,16α-dimethyl-17α-propionyloxy-1,4-pregnadien-3,20-dion.

5. Pharmazeutische Präparate gekennzeichnet durch einen Gehalt von ein oder zwei 6α,16α-Dimethylkortikoiden gemäß Patentanspruch 1 bis 4.

**Revendications**

1. Diméthyl-6α,16α corticostéroïdes répondant à la formule I:

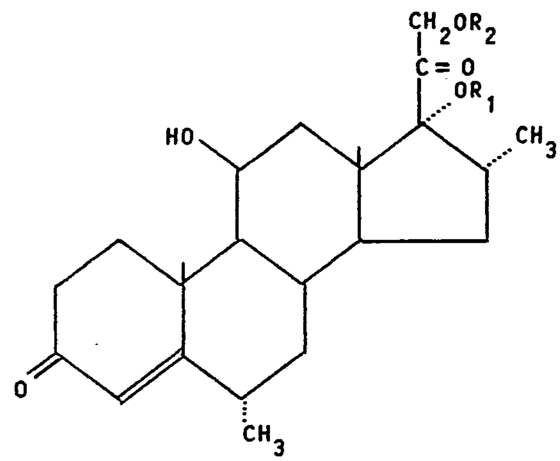

dans laquelle
la liaison . . . . . . représente une liaison simple ou une liaison double,
$R_1$ représente un radical alcanoyle contenant de 2 à 6 atomes de carbone et
$R_2$ représente un atome hydrogène ou un radical alcanoyle contenant de 2 à 6 atomes de carbone.

2. Dihydroxy-11β,21 diméthyl-6α,16α propionyloxy-17α prégnène-4 dione-3,20.

3. Acétoxy-21 hydroxy-11β diméthyl-6α,16α propionyloxy-17α prégnène-4 dione-3,20.

4. Acétoxy-21 hydroxy-11β diméthyl-6α,16α propionyloxy-17α prégnadiène-1,4 dione-3,20.

5. Compositions pharmaceutiques caractérisées en ce qu'elles renferment un ou deux diméthyl-6α,16α corticostéroïdes selon l'une quelconque des revendications 1 à 4.

**Claims**

1. 6α,16α-dimethyl corticoids of the general formula I

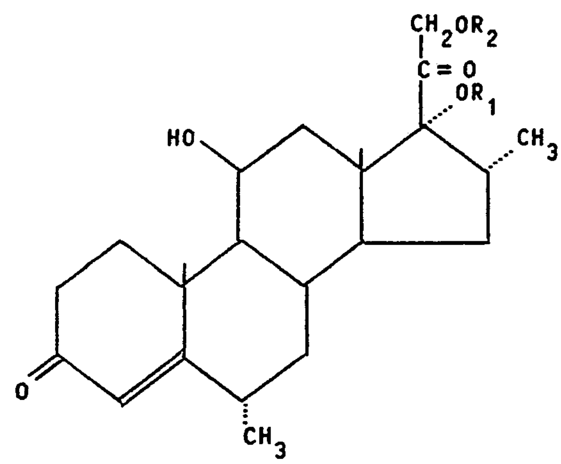

wherein
the bond . . . . . . is a single bond or a double bond,
$R_1$ represents an alkanoyl group containing from 2 to 6 carbon atoms and
$R_2$ represents a hydrogen atom or an alkanoyl group having from 2 to 6 carbon atoms.

2. 11β,21-dihydroxy-6α,16α-dimethyl-17α-propionyloxy-1,4-pregnene-3,20-dione.

3. 21-acetoxy-11β-hydroxy-6α,16α-dimethyl-17α-propionyloxy-4-pregnene-3,20-dione.

4. 21-acetoxy-11β-hydroxy-6α,16α-dimethyl-17α-propionyloxy-4-pregnadiene-3,20-dione.

5. Pharmaceutical preparations, characterised by a content of one or two 6α,16α-dimethyl corticoids according to patent claims 1 to 4.